# EUROPEAN PATENT SPECIFICATION

(11) **EP 1 461 012 B1**
(45) Date of publication and mention of the grant of the patent: **13.02.2008**
(21) Application number: 02797014.4
(22) Date of filing: 12.12.2002
(51) Int. Cl.: A61K 8/99, A61K 8/92, A61K 8/02, A61Q 17/00, A61K 35/74

(54) **HYGIENE TISSUE IMPREGNATED WITH A COMPOSITION COMPRISING A LACTIC PRODUCING BACTERIUM SUSPENDED IN A LIPID**
HYGIENEGEWEBE, IMPRÄGNIERT MIT EINEM MILCHSÄURE PRODUZIERENDEN BAKTERIUM, DAS IN EINEM LIPID SUSPENDIERT IST
SERVIETTE HYGIENIQUE IMPREGNEE D'UNE COMPOSITION CONTENANT UNE BACTERIE ENGENDRANT DE L'ACIDE LACTIQUE SUSPENDUE DANS UN LIPIDE

(30) Priority: 13.12.2001 SE 0104199
(43) Date of publication of application: 29.09.2004
(73) Proprietor: SCA Hygiene Products AB, 405 03 Göteborg (SE)
(72) Inventor: RUNEMAN, Bo, S-433 75 Partille (SE); FORSGREN-BRUSK, Ulla, S-435 43 Pixbo (SE); GRAN HÄKANSSON, Eva, S-907 40 Ume (SE)
(74) Representative: Larsson, Karin
(86) International application number: PCT/SE2002/002303
(87) International publication number: WO 2003/053397

(56) References cited:
- EP-A1- 1 118 342
- WO-A1-00/76878
- WO-A1-97/29762
- WO-A1-98/47374
- WO-A2-01/13956
- US-A- 4 518 696

## Description

The present invention relates to hygiene tissues to be used to reestablish and maintain a beneficial microbial flora on the skin and the urogenital area in combination with cleaning and caring of these areas.

### Background of the invention

The urogenital area harbors a complex microbial ecosystem comprising more than 50 different bacterial species (Hill et al., Scand. J. Urol. Nephrol. 1984;86 (suppl.) 23-29). The dominating species in this area are lactic acid producing bacteria belonging to the genus *Lactobacillus.* These lactic acid producing members are important for retaining a healthy microbial flora in these areas, and act as probiotic bacteria with an antagonistic effect against pathogenic microbial species. Lactic acid producing bacteria inhibit growth and colonization by other microorganisms by occupying suitable niches for colonization, by forming biofilms and competing for available nutrients, thereby excluding colonization by harmful microorganisms. Also, the production of enzymes, such as hydrogen peroxidase, and specific inhibiting substances, such as toxins and bacteriocines, and organic acids (including lactic acid and acetic acid) that lower the pH, inhibit colonization by other microorganisms. However, the microbial ecosystem of a healthy individual can be disturbed by the use of antibiotics, in people suffering from diabetes, during hormonal changes, such as during pregnancy or use of contraceptives with estrogen, during menstruation, after menopause, etc. Also, microorganisms can spread from the anus to the urogenital area, thereby causing infections. This results in a disturbance of the normal microbial flora and leaves the individual susceptible to microbial infections that cause vaginitis, urinary tract infections and ordinary skin infections. Microorganisms commonly associated with these kind of infections belong to the genera *Escherichia, Enterococcus, Psedomonas, Proteus, Klebsiella, Streptococcus, Staphylococcus, Gardnerella* and *Candida.* Women are at particular risk due to their shorter distance between the anus and the urogenital tract; specially at risk are young women, who not yet have a well developed microflora in the urogenital area and older women, who no longer have a protective flora.

Similarly to the urogenital area, the skin is colonized by an array of organisms, which forms its normal flora. The numbers and identity of the organisms vary between different skin sites. This, together with the skin's structural barrier, provides the host with an excellent defense against invading microbes. The number of bacteria on the skin vary from a few hundred per cm² on the arid surfaces of the forearm and back, to tens of thousands per cm² on the moist areas such as the axilla and groin. This normal flora plays an important role in preventing 'foreign' organisms from colonizing the skin, but it to needs to be kept in check, in order to avoid skin infections.

*Staphylococcus aureus* is the most common cause of minor skin infections, such as boils or abscesses, as well as more serious post-operative wound infection. Treatment involves drainage and this is usually sufficient for minor lesions, but antibiotics may be given in addition when the infection is severe and the patient has fever. Toxic shock syndrome is a systemic infection caused by *S*. *aureus* strains which produce toxic shock syndrome toxin. The disease came to prominence through its association with tampon use by healthy women, but it is not confined to women and can occur as a result of *S*. *aureus* infection at non-genital sites.

Other common skin infections are caused by *Streptococcus pyogenes* (group A streptococci). The organisms are acquired through contact with other people with infected skin lesions and may first colonize and multiply on normal skin prior to invasion through minor breaks of the epithelium and the development of lesions. Treatment with penicillin or erythromycin may be necessary to combat the infection.

*Propionibacterium acnes* are found on normal human skin. The organism is no longer believed to be the cause of acne, but have been assigned a role in inflammation of acne.

*Malassezia* (formerly *Pityrosporum)* are probably universal inhabitants of the head and thorax in adult humans. Species of this organism are known to be involved in the skin diseases seborrhoeic dermatitis and pityriasis versicolor and to play a part in the aetiology of severe dandruff. These yeasts may also play a part in exacerbation of atopic dermatitis.

So called ringworm infections of the skin may be caused by dermatophyte fungi, e.g. *Tricophyton, Epidermophyton* and *Microsporum.*

The relative dryness of most areas of skin limits the growth of *Candida,* which therefore are found in low numbers on healthy skin. However *Candida* rapidly colonizes damaged skin and intertriginous sites (apposed skin sites which are moist and become chafed). *Candida* also colonizes the oral and vaginal mucosa and overgrowth may result in disease in these sites (so called thrush). *C. albicans* is associated with diaper dermatitis. A study has shown that *C. albicans* induced lesions are remarkably influenced by pH, a lower skin pH giving less lesions (B. Runeman, Acta Derm Venereol 2000; 80: 421-424).

One way to reduce the problems with the kind of infections described above is to have a good personal hygiene. However, excessive use of cleaning agents not only decrease the amount of harmful microbes, but can harm the beneficial microbial flora, again render it susceptible for pathogenic species to colonize and cause infections. Alternatively, administration of lactic acid producing bacteria to the urogenital area and the skin in order to outcompete pathogenic species and facilitating reestablishment and maintenance of a beneficial microbial flora in these areas, have been found to be a successful means to treat and prevent microbial infections.

It has been suggested that lactic acid producing bacteria can be delivered via absorbent articles, such as diapers, sanitary napkins, panty liners and tampons, as described in, for example, in WO97/02846, WO99/17813, WO99/45099 and WO00/35502. However, absorbent articles may not always be an optimal administration route, since carrying of an absorbent article often is apprehended as uncomfortable, indiscrete and warm. This administration route can also be inconvenient as repeated administration of lactic acid producing bacteria often is necessary to retain the efficacy of the treatment or the preventative effect. Also, these products cannot be used for delivery of the bacteria to other regions of the body than the urogenital area. Therefore, for some applications it can be more convenient to administer lactic acid producing bacteria by other means than absorbent products. A second problem with administration of lactic acid producing bacteria via absorbent articles relates to the manufacturing of such products, since all possible variants and sizes of the product have to be supplied with the bacteria. Therefore the administration via a product that could be used without individual adjustments could provide a manufacturing advantage over the absorbent products.

However, a major problem with providing articles intended to be used for transfer of lactic acid producing bacteria, is that the bacteria have to retain viability during transport and storage of the articles. Lactic acid producing bacteria rapidly lose viability under moist conditions, and it is therefore important that the products are not exposed to moisture. One way to partly overcome this problem has been to supply articles with freeze-dried lactic acid producing bacteria, thereby providing long shelf-life products containing viable lactic acid producing bacteria. However, the bacteria still have to be protected against moisture during the time between manufacturing and use.

Alternatively, research experiments have shown that storage in sterile vaseline oil results in a high level of viable lactobacilli cells after 8 months of storage, although survival of the bacterial cells is not discussed in the context of transferring bacteria to the skin (Arkadéva et al., N A. Nauchnye Doklady Vysshei Shkoly. Biologiches-kie Nauki, 1983, 2:101-104). In contrast, Stoianova et al. (Mikrobiologiia, 2000, 69:98-104) found that immersion in mineral oil was not effective to preserve viability of lactic acid producing bacteria. There are additional examples of the combination lactic acid producing bacteria and a fatty composition, although these do not describe the effect of the fatty composition on the survival. WO01/13956 describes pharmaceutical compositions comprising Emu oil, antimicrobial agents and/or *Bacillus coagulans* to be used for antimicrobial treatments. However, the object of using compositions described in WO01/13956 is to treat microbial infections by adding components that kill undesirable microorganisms and the Emu oil is not added to enhance survival of bacteria included in the compositions. WO92/13577 relates to a tampon or sanitary napkin that is coated with a compound with adhesive properties and subsequently added bacteria that attach to the adhesive compound.
WO92/13577 neither relates to hygiene tissues nor mentions anything about a composition containing bacteria suspended in a lipid phase. US 4,518,696 describes that suspensions comprising lactobacilli can be stabilised by dispersing the bacteria in sun flower oil. However, US 4,518,696 relates to the field of providing preparations of lactobacilli for oral administration to animals.

In conclusion, there is still a need to develop products for delivery of lactic acid producing bacteria to the skin and urogenital area that are convenient to use, result in efficient transfer of the bacteria to the area where they are applied and can be stored for long time periods without loss of viability of the bacterial cells.

### Object of the invention

The object of the present invention is to provide a convenient device for the delivery of lactic acid producing bacteria to the skin and urogenital area. This is obtained by providing a hygiene tissue, comprising viable lactic acid producing bacteria, that can be used for cleaning and caring the skin while simultaneously delivering the lactic acid producing bacteria, to reestablish and maintain a beneficial microflora on the skin and the urogenital area. In order to provide products that can be stored for long time periods, without loss of viability of the lactic acid producing bacteria, the bacterial cells are suspended in a lipid that protects the bacteria from moisture. Also an object of the present invention is to provide moisture impervious packing units comprising the hygiene tissue of the invention.

### Summary of the invention

The present invention pertains to a hygiene tissue to be used for cleaning and caring of the skin and the urogenital area simultaneously as it delivers lactic acid producing bacteria, thereby reestablishing and maintaining a healthy microbial flora in these areas. The hygiene tissue is characterized in that it is impregnated with a composition comprising a lactic acid producing bacterium/bacteria suspended in a specific lipid phase and optionally (an) additional components. The present inventors surprisingly found that encapsulating the lactic acid bacterium in a specific lipid provided a moisturefree environment keeping the bacterium in a shape that resulted in enhanced longevity, high transfer rates to the skin, and still keeping fitness for survival and growth on the skin. Therefore, by this approach, bacterial survival was enhanced during long term storage. Also, the hygiene tissue of the present invention improved the efficiency of transfer of the lactic acid producing bacterium to the skin and urogenital area, simultaneously as the lipid served as a cleaning agent with skin caring properties. Furthermore, the invention relates to an impervious packing unit comprising the hygiene tissue described above.

### Short description of drawings

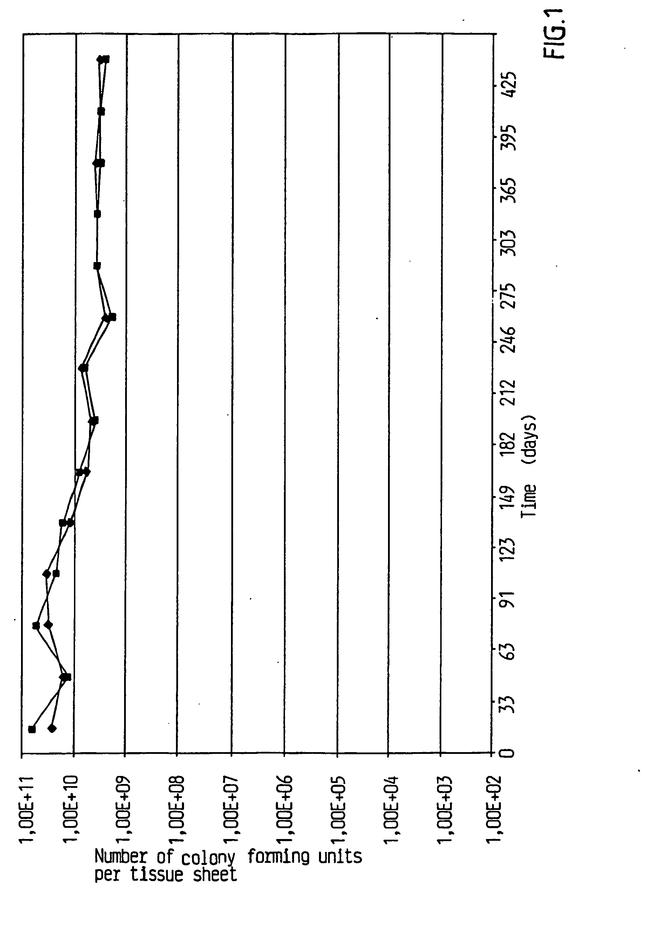

In all figures 1.00E+02 is 1.00x10², 1.00E+03 is 1.00x10³, etc..

Figure 1 shows the survival of *Lactobacillus plantarum* 931 in olive oil on hygiene tissues Spun Lace Dupont (◆) and SCA Absbond (■).

Figure 2 shows the survival of *Lactobacillus plantarum* 931 in suspensions of water and olive oil on hygiene tissues (Spun Lace Dupont). (◆) 10% olive oil in water, (■) 30% olive oil in water.

Figure 3 shows the survival of *Lactobacillus plantarum* 931 in olive (◆) and rapeseed (■) oil.

Figure 4 shows the survival of *Lactobacillus plantarum* 931 in lipids with different chemical compositions. (◆) vaseline, (■) paraffin, (▲) glycerolum, (×) olive oil, (*) Dimeticonum, (•) Akoline MCM, (↓) Akomed R, (⁻) Akorex L.

Figure 5 shows the survival of *Lactobacillus plantarum* 931 in suspensions of water and olive oil. (•) 10% olive oil in water and (■) 30% olive oil in water.

Figure 6 shows transfer efficacy to and survival rate on skin of *Lactobacillus plantarum* 931 suspended in olive oil on hygiene tissues used on five different subjects.

Figure 7 shows transfer efficacy to and survival rate on skin of *Lactobacillus plantarum* 931 suspended in paraffin oil on hygiene tissues used on five different subjects.

Figure 8 shows transfer efficacy to and survival rate on skin of *Lactobacillus plantarum* 931 suspended in Milli Q water on hygiene tissues used on five different subjects.

Figure 9 shows transfer to and survival of *Lactobacillus plantarum* 931 suspended in olive oil in the urethra after application via a tissue sheet used in the urogenital area on four different subjects.

Figure 10 shows transfer to and survival of *Lactobacillus plantarum* 931 suspended in olive oil in the perineum after application via a tissue sheet used in the urogenital area on four different subjects.

Figure 11 shows the growth rate of *Lactobacillus plantarum* 931 cells after storage in olive oil on a tissue sheet (Δ and o) as compared to the growth rate of freshly inoculated *L. plantarum* 931 cells from an overnight culture (■).

### Definitions

By "hygiene tissue" is meant any device for wiping skin, for instance, a washcloth, patch, towelette, napkin, wetwipe, and the like.

By "matrix" is meant any natural or synthetic fiber, such as felt, batting, rayon, cellulose, regenerated cellulose, polyester, polyolefin fibers, textile and the like, or foam.

Preferred "lactic acid producing bacterial strain" for the object of the present invention include bacteria from the genera *Lactobacillus, Lactococcus* and *Pediococcus.* Preferably the selected bacterium used is from the species *Lactococcus lactis, Lactobacillus acidophilus, Lactobacillus curvatus* or *Lactobacillus plantarum.* Even more preferably the lactic acid producing bacterium is *Lactobacillus plantarum* 931 (deposition No. (DSM): 11918).

By "additional component" is meant agents commonly added to skin caring products, such as caring agents, water absorbent agents, pH buffering agents (weak organic or inorganic acids, such as lactic acid, ascorbic acid, citric acid or boric acid), perfume, antioxidants, hydrocortisone, other anti-inflammatory steroids, etc. Further details on suitable agents commonly added to skin caring products are given in Harry's Cosmeticology 8th ed., Ed by MM Rieger, Chemical Publishing Co., Inc., New York, 2000.

By "moisture impervious packing unit" is meant a packing unit having a highest water vapor transmission rate of 6 g/m²/calendar day in accordance with ASTME 398-83.

### Detailed description of the invention

The hygiene tissue provided in the present invention is intended to be used for cleaning and simultaneously reestablishing and maintaining a healthy microbial flora on the skin and in the urogenital area. The hygiene tissue provided can be composed of a matrix comprising any natural or synthetic fiber, such as felt, batting, rayon, cellulose, regenerated cellulose, polyester, polyolefin fibers, textile and the like, or foam, or combinations thereof. The water content of the matrix of the hygiene tissue is preferably 10% or less (by weight), more preferably 5% or less (by weight) and most preferably 1% or less (by weight). The hygiene tissue is impregnated with a suspension of a lactic acid producing bacterium in a lipid. The lipid encapsulates the bacteria and thereby acts to protect the bacteria from moisture, which enhances bacterial survival during manufacturing and storage. Exposure to moisture during manufacturing and storage of products that comprise lactic acid producing bacteria, causes reactivation of the bacteria, which subsequently leads to their death. Therefore, by protecting the bacteria from moisture, their survival is enhanced and the durability of the product extended. The inventors also found that encapsulating the bacteria in lipid resulted in enhanced transfer rates to and survival of the bacteria on the skin after their delivery to it (see Example 3 below). This can be an effect of the lipid creating a micromilieu, that is beneficial for retaining bacterial viability and that enhances growth of the added bacteria on the skin. Also, the lipid has more adhesive properties than, for example, water, thereby resulting in a higher amount of bacteria actually being transferred to the skin. In addition to its bacterial survival enhancing properties, the lipid also serves as a skin treatment agent that cleans the skin without causing drying out. Once the bacteria have been delivered to the skin, the moist on the skin reactivates the bacteria, thereby allowing them to perform their intended action, i.e. competitively exclude and prevent colonization of pathogenic microbial species. Optionally, additional substances, including water absorbent agents (such as inorganic salts, e.g. calcium chloride), tensides (non-ionic, amphoteric and anionic surfactants), pH buffering agents (weak organic or inorganic acids, such as lactic acid, ascorbic acid, citric acid or boric acid), perfume, antioxidants, hydrocortisone and other anti-inflammatory steroids, can also be added to the lipid-bacterial suspension, or directly to the hygiene tissue.

The amount of lipid suspension of lactic acid producing bacteria on the tissue is 0.5-95% by weight.

The number of probiotic bacteria on the hygiene tissue is preferably 10⁴-10¹¹ colony forming units (CFU) and more preferably 10⁶-10¹¹ CFU. The preparation of lactic acid producing bacteria are preferably provided in a dried form, preferably as a freeze-dried powder. The bacteria can also be provided in microincapsulated forms. The water activity of the bacterial preparation is 0.30 or less, more preferably 0.25 or less, most preferably 0.20 or less.

In one preferred embodiment the probiotic lactic acid producing bacterial strain with antagonistic effect is selected from the genera *Pediococcus, Lactobacillus* or *Lactococcus* including combinations thereof. The lactic acid producing bacterial strain is preferably isolated from the skin or urogenital area of a healthy person.

In another preferred embodiment the probiotic bacterial strain with antagonistic effect is at least a *Lactobacillus plantarum* strain.

In an even more preferred embodiment the probiotic bacterial strain with antagonistic effect is at least *Lactobacillus plantarum* 931 (deposition No. (DSM): 11918).

Suitable lipids for the present invention support survival of the stored cells of so that the maximum decrease in number of culturable cells is 3 log units after 12 months storage. Suitable lipids for the present invention preferably have a water content of 5% or less (by weight), preferably 3% or less (by weight), most preferably 1% or less (by weight).

More preferably, suitable lipids support survival of the stored cells of so that the maximum decrease in number of culturable cells is 2 log units after 12 months storage.

Most preferably, suitable lipids support survival of the stored cells of so that the maximum decrease in number of culturable cells is 1 log unit after 12 months storage.

Suitable lipids enable transfer of bacteria to the skin of more 10⁵ or more culturable cells per cm².

More preferably, suitable lipids enable transfer of bacteria to the skin of 10⁶ or more culturable cells per cm².

Most preferably, suitable lipids enable transfer of bacteria to the skin of 10⁷ or more culturable cells per cm².

Suitable lipids are also characterized by supporting survival of the bacterial cells on the skin so that 10² or more culturable cells per cm² can be recovered 12 hours after delivery by use of the hygiene tissue.

More preferably, suitable lipids support survival of the bacterial cells on the skin so that 10³ or more culturable cells per cm² can be recovered 12 hours after delivery by use of the hygiene tissue.

Most preferably, suitable lipids support survival of the bacterial cells on the skin so that 10⁴ or more culturable cells per cm² can be recovered 12 hours after delivery by use of the hygiene tissue.

Preferably a liquid lipid is used in the present invention.

Lipids of the invention include olive oil, canola oil, coconut oil, palm kernel oil, peanut oil, soy bean oil, Dimethicone, paraffin oil, and petrolatum. These lipids provide high survival of lactic acid bacteria and high transfer rates of the bacteria to the skin and urogenital area. In addition these lipids have positive effects in the skin; they have a soothing effect, show skin protective properties and are non-toxic and non-allergenic. The preferred lipids are all of non-animal origin.

In order to protect the probiotic bacteria from moisture during storage and transportation, the hygiene tissue is preferably provided individually packed in a moisture impervious packing unit. The packing unit can be constructed in several different ways. Importantly, the material used should not have a vapor permeability exceeding 6g/m²/calendar day in accordance with ASTME 398-83 at 37.8°C and 90% relative humidity. Suitable materials to be used to obtain an impervious packing unit include polyethylene, polypropylene, polyester, polyamide, polyvinylalcohol and similar polymers, aluminum foil, aluminum oxide, silicon oxide, and the like. In order to produce packing material that has good wear strength and can readily be sealed, a less expensive material may be used as outer protective wear layers and/or as inner sealing layers. For instance, the packing unit can comprise an inner material that enables a good seal to be obtained, e.g. polyethylene, polypropylene, butyl acrylate, vinyl acetate or wax, an intermediate material that consists of a moisture impervious material, and a stronger outer barrier material, e.g. polyethylene or polypropylene. After placement of the hygiene tissue comprising the lipid suspension containing the lactic acid producing bacteria, all openings of the packing unit have to be tightly sealed. It is important that the seals do not have a moisture permeability exceeding that of the packing material itself. Further details on the design of suitable impervious packing units are given in WO00/76878, which hereby is incorporated as reference.

### Example 1

### Survival of Lactobacillus plantarum 931 in olive oil on hygiene tissues

A preparation of freeze dried *L. plantarum* 931 cells in skimmilk was grounded until a powder of fine grains was formed. 10 g of the *L. plantarum* 931 powder was added to 120 ml olive oil (Filippo BERIO extra virgin olive oil) and shaken until a homogenous solution was formed. An additional aliquot of 80 ml of olive oil was added and the resulting 200 ml solution was vortexed for ca 2 min. The bacterial suspension was kept at room temperature for 3 hours, with mixing twice an hour. Tissue sheets (Spun Lace Dupont and SCA Absbond) were cut to 6x4 cm squares and placed in sterile stainless steel trays. On each tissue sheet, 2 ml of bacterial suspension was dropped over the tissue to cover it. The tissue sheet was folded in the middle, then from the long side to the middle again and packed in foil bags, which edges were welded. Samples were removed for determination of initial bacterial concentration and the remaining bags were stored at room temperature for viability studies.

As a comparison to storage of *L. plantarum* 931 cells in olive oil, *L. plantarum* 931 cells were stored in suspensions of water and olive oil on hygiene tissues. These were prepared by mixing vigorously 2.5 g of freeze dried *L. plantarum* 931 cells (prepared as described above) with 45 ml of Milli Q water and 5 ml olive oil (Filippo BERIO, Italy) or 35 ml Milli Q water and 15 ml olive oil to prepare suspensions with approximately 10 or 30% oil, respectively. The bacterial oil-water suspensions were applied to tissue sheets (Spun Lace Dupont) for bacterial survival studies. On each tissue sheet (6x7 cm), 1 ml of bacterial suspension was dropped to cover the tissue. The tissue sheet was folded in the middle, then from the long side to the middle again and packed in foil bags, which edges were welded. Samples were removed for determination of initial bacterial concentration and the remaining bags were stored at room temperature in the dark for viability studies.

To test the viability of the *L. plantarum* 931 cells after storage for different time periods on a tissue sheet, the tissue sheet was transferred to a Stomacher bag and 10 ml of 0.9% NaCl was spread over the tissue sheet. The bag was run for 3 min on high effect in Stomacher. The contents of the bag was then transferred to test tubes, diluted in 0.9% NaCl when necessary, and immediately plated onto Rogosa plates. The number of colonies was counted after 2 days of incubation at 37°C in 5% CO₂ in air. Two tissue sheets were analyzed at each sampling date.

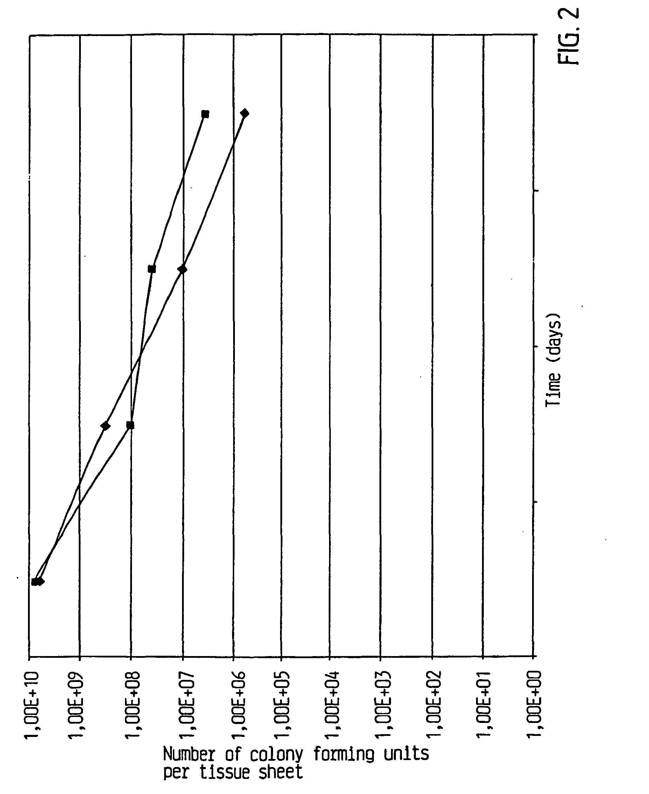

The survival of *L. plantarum* 931 in olive oil on hygiene tissues for a total time period of one year and three months is shown in Fig. 1. The survival of the *L. plantarum* 931 cells stored under these conditions was very high for both tissue sheets variants tested. In comparison, storage of oil-water suspensions (10 and 30% olive oil in water) of the bacteria on a tissue sheet (Fig. 2) resulted in a rapid decrease in viability with a drop of more than 10³ orders of magnitude over just a three months time period studied.

### Example 2

### Survival of L. plantarum 931 in lipids with different chemical compositions

497 mg of a powder of *L. plantarum* 931 in skim milk, prepared as described in Example 1, was mixed with 5 ml of olive oil (Filippo BERIO extra virgin olive oil, Filippo BERIO, Italy) or rapeseed oil (Felix AB, Sweden). The pH of the oils was ca 5. The rapeseed oil also contained, in addition to rapeseed oil, citric acid and vitamin A and D. The suspensions were vortexed for 1 min and allowed to rest for 1 min. This was repeated four more times. The bacterial suspensions were kept for 4 hours at room temperature with mixing twice an hour. The suspensions were then divided into 1 ml aliquots and stored in sterile brown glass vials. The initial concentrations of bacteria in the suspensions were determined. The vials were stored in a dark place at room temperature and normal air humidity varying from 30-60%.

In addition to the experiment described above, to further compare the survival of *L*. *plantarum* 931 in lipids with different compositions, 2 g of freeze-dried *L. plantarum* 931 cells in skimmilk (2x10¹⁰ colony forming units/g) were mixed with either 40 ml or 40 g of the different lipid compositions, depending on the lipid consistency. The tested lipids were: white vaseline (petrolatum, Apoteket AB, Umeå, Sweden), paraffin (paraffinum liquidum, Apoteket AB, Gothenburg, Sweden), glycerol ("glycerin", maximum water content 0.5%, Apoteket AB, Gothenburg, Sweden), olive oil ("Olea Europea", cold pressed, Apoteket AB, Gothenburg, Sweden), dimethiconum (Dimethicone, 350 cSt., Dow Coming 200/350 S fluid, Kebo Lab, Sweden), and Akoline MCM (mono-diglyceride of medium chain fatty acids; primarly caprylic and capric acids, , Karlshamns AB, Sweden), Akomed R (caprylic/capric triglyceride from coconut and/or palm kernel oils, deodorized, Karlshamns AB, Sweden), and Akorex L (canola oil, partially hydrogenated, deodorized, Karlshamns AB, Sweden). The samples were stored in sterile, brown, glass vials at room temperature in the dark at normal air humidity (varying from 30-60% relative humidity). To establish the number of viable *L. plantarum* 931 cells after different storage times, 1 g of the samples was transferred to a stomacher bag and 9 ml of 0.9% NaCl was added. The bag was then run at high effect in Stomacher for 3 min. The contents of the bag was transferred to test tubes, diluted when necessary in NaCl and cultured on MRS-plates at 37°C in 5% CO₂ in air for 2 days.

As a comparison to storage *of L. plantarum* 931 cells in different lipids, *L. plantarum* 931 cells were also stored in suspensions of water and olive oil. These were prepared by mixing vigorously 2.5 g of freeze dried *L. plantarum* 931 cells (prepared as described above) with 45 ml of Milli Q water and 5 ml olive oil (Filippo BERIO, Italy) or 35 ml Milli Q water and 15 ml olive oil to prepare suspensions with approximately 10 or 30% oil, respectively. The samples were stored in sterile plastic vials at room temperature at normal air humidity (varying from 30-60% relative humidity). To test survival after different time intervals, samples were removed from the vials, diluted in 0.9% NaCl and incubated at 37°C in 5% CO₂ in air for 2 days.

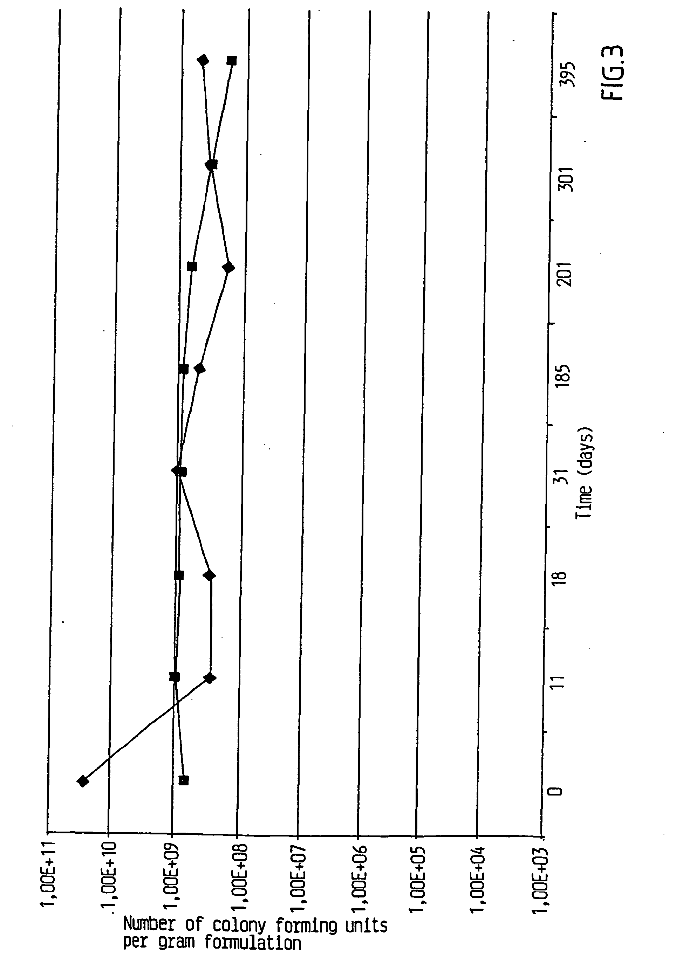

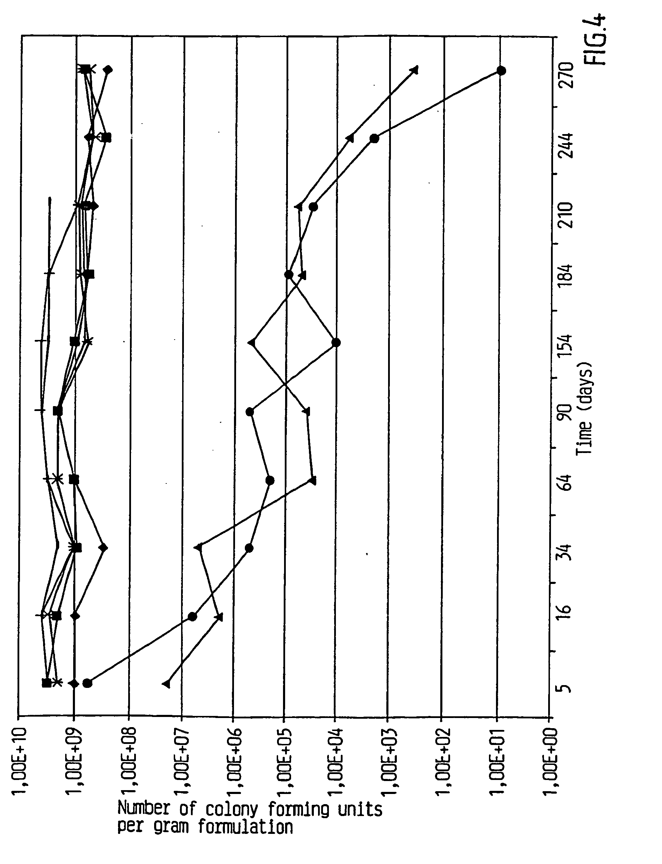

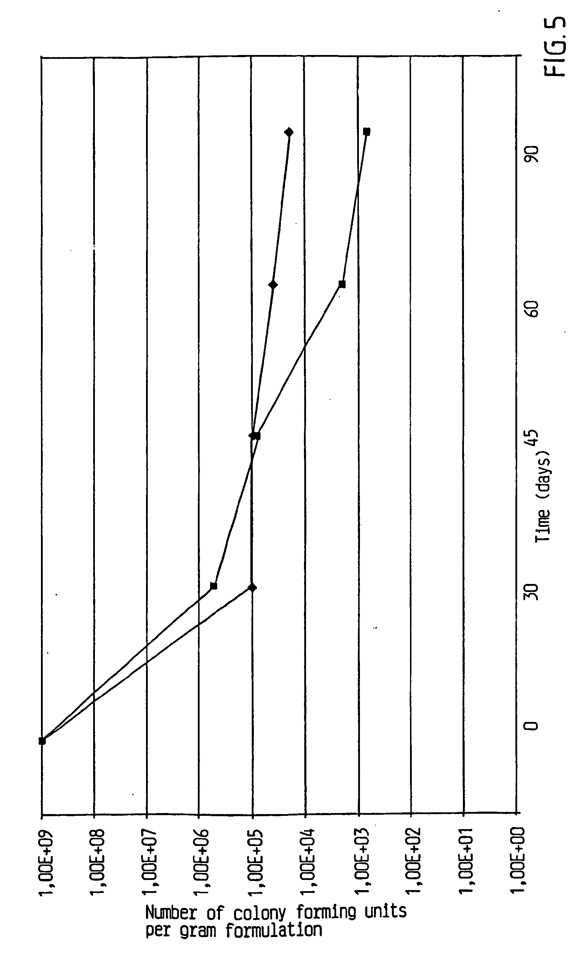

Figure 3 shows an extraordinarily high level of survival of the *L. plantarum* 931 cells in olive and rapeseed oil over the more than one year time period studied. Storage in vaseline, paraffin, Dimeticonum, Akoline MCM, Akomed R, and Akorex L also resulted in a high survival over extended time periods (Fig. 4). However, storage in the more hydrophilic glycerol resulted in a pronounced decrease in survival over time (Fig. 4). Also, the Akoline MCM, known to have bacteriostatic properties, could not support survival of the cells (Fig. 4). In addition, it can be seen that storage in the more hydrophilic environment provided in the oil-water suspensions (Fig. 5) results in a very rapid initial decrease in viability by more than 10² orders of magnitude over the first month of storage. During the next two months studied, the decrease in survival rates flattened out, but still the decrease in survival is much higher than what is observed when the bacterial cells were stored in a lipid.

### Example 3

### Study on transfer efficacy to and survival rates on skin of L. plantarum 931 suspended in olive oil, paraffin oil or Milli Q water

2.00 g of freeze-dried *L. plantarum* 931 were added to a sterile glass vial and 40 ml of olive oil, paraffin oil or Milli Q water were added. The suspensions were shaken until homogenous solutions formed and were left at room temperature for four hours. Tissue sheets with *L. plantarum* 931 were prepared by cutting tissue sheets to 7x8 cm pieces and dropping 2 ml of the different bacterial suspensions, prepared as described above, to cover the tissue. The tissue sheets were folded in the middle, then from the long side to the middle again and packed in foil bags which edges were welded. Samples were removed for determination of initial bacterial concentrations and the remaining bags were stored at room temperature for viability studies. Two of the prepared tissue sheets with *L. plantarum* 931 for each preparation were used in the bend of the arm on five subjects (i.e. one tissue was used for three subjects and the other one for two). Before the test, samples were taken to assure that no *L. plantarum* 931 cells were initially present on the skin. On each subject, in one bend of the arm a tissue sheet with *L. plantarum* 931 in Milli Q water was streaked and a tissue sheet with *L. plantarum* 931 in olive oil was similarly used in the other. The skin was then sampled for presence *of L. plantarum* 931 cells at 0, 4, 6 and 24 hours after streaking. The sampling procedure was as follows: a sterile stick provided with a cotton wool top was dipped in 0.9% NaCl and rolled 4 times over an area of 1 cm² at the site of application of the bacteria. The stick was then dipped in 1 ml of 0.9% NaCl and mixed. The samples were diluted in 0.9% NaCl and immediately plated onto Rogosa plates. The plates were incubated at 37°C in 5% CO₂ in air for 2 days. After 24 hours the bend of the arm, where *L. plantarum* 931 cells in Milli Q water were applied, was rinsed with Sumabac (Diversey Lever, Huddinge, Sweden) and a tissue sheet with *L. plantarum* 931 cells in paraffin oil was streaked in that bend of the arm, which, as previously, was sampled at 0, 4, 6 and 24 hours after streaking to assess the amount of *L. plantarum* 931 cells on the skin.

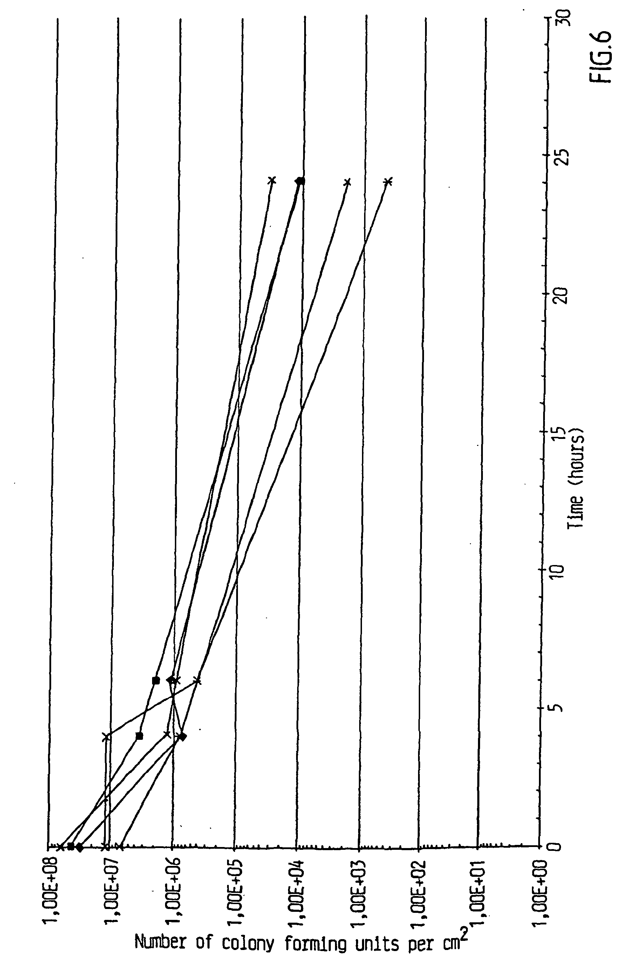

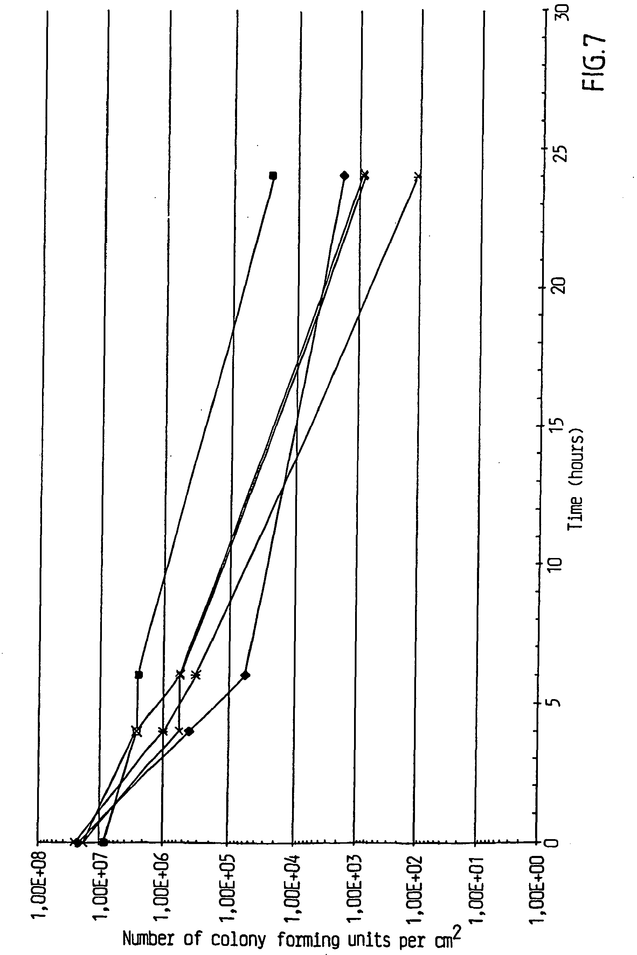

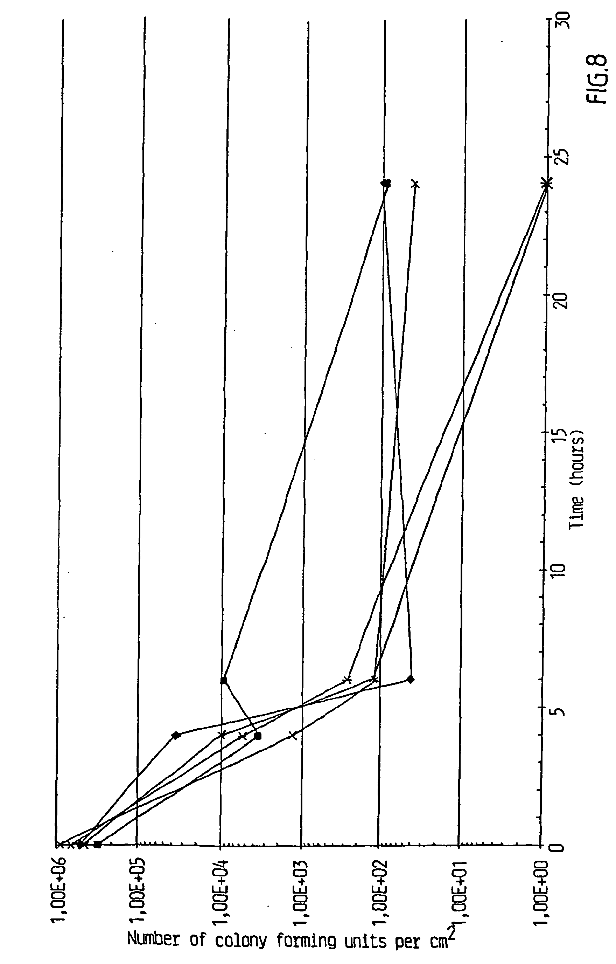

As shown in Fig. 6-8 the amount *of L. plantarum* 931 cells transferred to the skin was usually more than 10 times higher when the bacteria were suspended in olive (Fig. 6) or paraffin oil (Fig. 7) compared to when they were suspended in Milli Q water (Fig. 8). Suspending the bacteria in lipid instead of water therefore was demostrated to enhance the transfer rate of the bacteria to the skin. Also, once the *L*. *plantarum* 931 cells were on the skin, the lipid also enhanced bacterial survival, since the initial drop in bacterial counts was slower and the final level of bacteria on the skin after 24 hours was much higher after use of a tissue where the bacteria were suspended in olive or paraffin oil (Fig. 6 and 7), compared to what was found when water was used as suspending agent (Fig. 8).

### Example 4

### Transfer of L. plantarum 931 suspended in olive oil applied to a tissue sheet used in the urogenital area

A bacterial suspension was prepared by adding 5.051 g of a powder of *L. plantarum* 931 (prepared as described in Example 1) to 100 ml olive oil (Filippo BERIO extra virgin olive oil). The powder was first added to 75 ml of olive oil and shaken to form a homogenous solution before addition of another 25 ml, followed by vortex-ing for 2 min. The bacterial suspension was kept at room temperature for 2 hours, with mixing twice an hour. A tissue sheet was prepared and inoculated with the bacteria as described in Example 1. 4 girls were treated with the tissue sheet in the urogenital area. Samples were collected from the urethra and perineum, using sterile cotton sticks, before using the tissue sheet, to ensure that no lactobacilli were initially present. Thereafter samples were collected immediately after treatment, and after 2, 4, 6, and 17 hours, to monitor transfer and survival of the lactobacilli by dipping a sterile stick provided with a cotton wool top in MRS-broth and rolling it three times over an area of 1 cm² at the site of application of the bacteria. The stick was then put in one ml MRS-broth. In the same way a stick was rolled over the uretra over an area of ¼ cm² and was the put in another tube of MRS-broth . The samples were plated onto Rogosa plates containing 128 µg/ml vancomycin. The plates were incubated at 37°C in 5% CO₂ in air for two days.

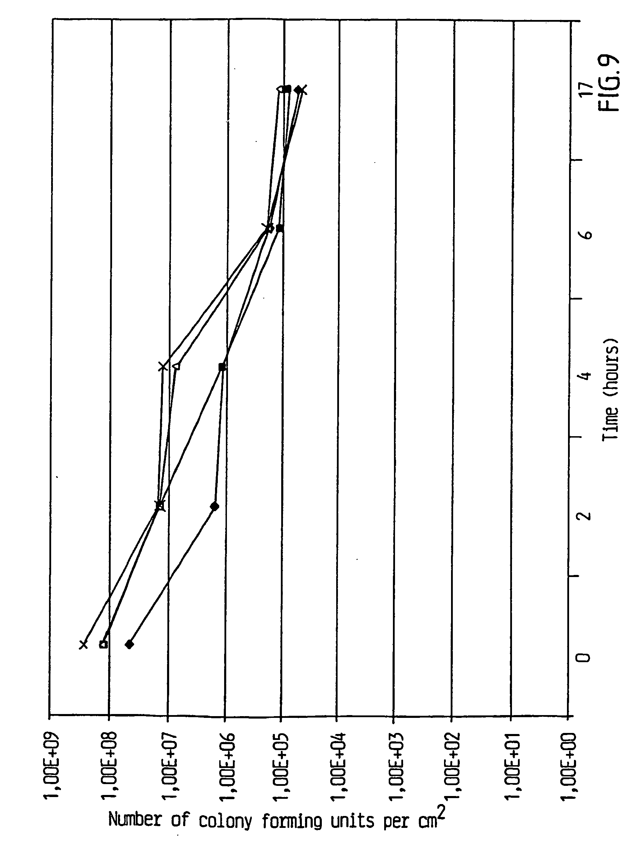

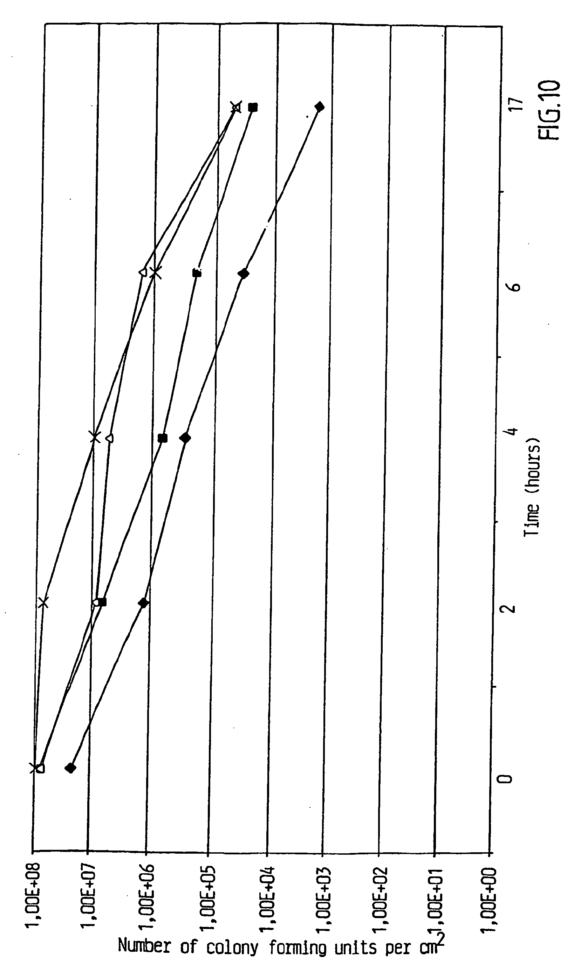

The girls were not allowed to take a bath or shower during the study, but were not given instructions in any other respect. The results shown in Fig. 9 and 10 demonstrate that there was a high level of transfer of *L. plantarum* 931 to the urethra (Fig. 9) and perineum (Fig. 10) and that a surprisingly high amount of bacteria remained in these areas during the time period studied.

### Example 5

### Viability of L. plantarum 931 after storage in olive oil on a hygiene tissue

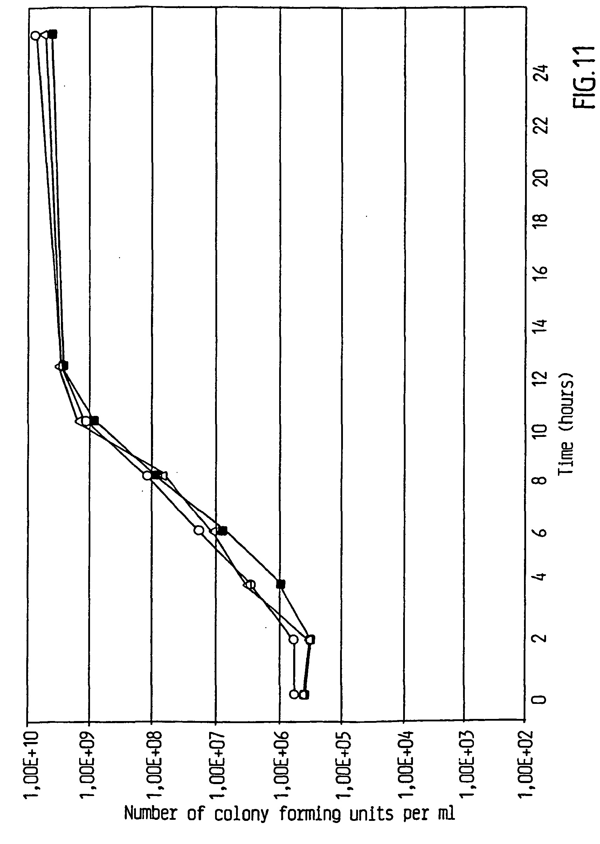

Growth *of L. plantarum* 931 suspended in olive oil on tissue sheets that had been stored for 6 days was compared to growth of *L. plantarum* 931 that were not suspended and stored in olive oil. The tissue sheets were wetted with 10 ml 0.9% NaCl and run in Stomacher for 3 minutes on high effect. The solution was then transferred to test tubes and 10 µl were inoculated into 10 ml MRS broth. The bacterial concentration was followed during growth at 37°C in 5% CO₂ in air by quantifying the number of colony forming units (CFU) and the optical density (OD) at 0, 2, 4, 6, 8, 10, 12 and 24 hours. Duplicate samples were taken for the analysis. The results shown in Fig. 11 demonstrate that the capability of growth of the *L. plantarum* 931 cells had been suspended and stored in olive oil on a tissue sheet is just as good as growth of cells from an overnight culture *of L. plantarum* 931. Accordingly, the *L. plantarum* 931 cells were not negatively affected by the storage in olive oil.

## Claims

1. Hygiene tissue suitable for cleaning and simultaneously reestablishing and maintaining a beneficial microbial flora on the skin or the urogenital area, said hygiene tissue comprising a matrix **characterized in that** said matrix is impregnated by a composition consisting of
A) a preparation of one or more lactic acid producing bacterial strains, said preparation having a water activity of 0.30 or less, more preferably 0.25 or less, most preferably 0.20 or less; and
B) a lipid phase, preferably a liquid lipid phase, comprising at least one lipid wherein the lipid is chosen from the group consisting of olive oil, canola oil, coconut oil, palm kernel oil, peanut oil, soy bean oil, Dimethicone, paraffin oil, and petrolatum.

2. Hygiene tissue according to claim 1 which further comprises at least one additional component, chosen from the group comprising caring agents, water absorbent agents, tensides, pH buffering agents, perfume, antioxidants, hydrocortisone and other anti-inflammatory steroids.

3. Hygiene tissue according to claim 1-2 where the lactic acid producing bacterial strain(s) is isolated from the skin or urogenital area of a healthy person

4. The hygiene tissue of claim 1-3, where the lactic acid producing bacterial strain(s) is selected from the genera *Pediococcus, Lactococcus, Lactobacillus,* or mixes thereof.

5. The hygiene tissue of claim 4, where the lactic acid producing bacterial strain(s) involves at least *Lactobacillus plantarum.*

6. The hygiene tissue of claim 5, where the lactic acid producing bacterial strain(s) involves at least *Lactobacillus plantarum* 931 (deposition No. (DSM): 11918).

7. A hygiene tissue according to claims 1-6, wherein the amount of lactic acid producing bacteria are 10⁴-10¹¹ colony forming units (CFU) per tissue.

8. A hygiene tissue according to claim 7, wherein the amount of lactic acid producing bacteria are 10⁶-10¹¹ colony forming units (CFU) per tissue.

9. A moisture impervious packaging unit containing a hygiene tissue according to claim 1-8, said impervious packing unit having a highest water vapor transmission rate of 6 g/m²/calendar day in accordance with ASTME 398-83.

## Patentansprüche

1. Reinigungstuch, geeignet zum Reinigen und gleichzeitigen Wiederherstellen und Beibehalten einer zuträglichen mikrobiellen Flora auf der Haut oder dem Urogenitalbereich, wobei das Hygienetuch eine Matrix umfaßt, **dadurch gekennzeichnet, daß** die Matrix mit einer Zusammensetzung imprägniert ist bestehend aus
A) einer Zubereitung von einem oder mehreren Milchsäure-produzierenden Bakterienstamm/stämmen, wobei die Zubereitung eine Wasseraktivität von 0,30 oder kleiner, stärker bevorzugt 0,25 oder kleiner, am stärksten bevorzugt 0,20 oder kleiner, aufweist; und
B) einer Lipidphase, bevorzugt einer flüssigen Lipidphase, die zumindest ein Lipid umfaßt, worin das Lipid ausgewählt ist aus der Gruppe bestehend aus Olivenöl, Rapsöl, Kokosnußöl, Palmkernöl, Erdnußöl, Sojabohnenöl, Dimethicon, Paraffinöl und Petrolatum.

2. Reinigungstuch gemäß Anspruch 1, welches ferner zumindest eine zusätzliche Komponente umfaßt, ausgewählt aus der Gruppe umfaßend Pflegemitteln, wasserabsorbierenden Mitteln, Tensiden, pH-Puffermitteln, Parfüm, Antioxidantien, Hydrocortison und anderen entzündungshemmenden Steroiden.

3. Reinigungstuch gemäß Anspruch 1 bis 2, worin der/die Milchsäure-produzierenden Bakterienstamm/stämme aus der Haut oder dem Urogenitalbereich einer gesunden Person isoliert ist/sind.

4. Reinigungstuch gemäß Anspruch 1 bis 3, worin die/der Milchsäure-produzierende(n) Bakterienstamm/stämme aus den Gattungen *Pediococcus, Lactococcus, Lactobacillus* oder Mischungen hiervon ausgewählt ist/sind.

5. Reinigungstuch gemäß Anspruch 4, worin der/die Milchsäure-produzierende(n) Bakterienstamm/stämme zumindest *Lactobacillus plantarum* beinhalten.

6. Reinigungstuch gemäß Anspruch 5, worin der/die Milchsäure-produzierende(n) Bakterienstamm/stämme zumindest *Lactobacillus plantarum* 931 (Hinterlegungsnr. (DSM): 11918) beinhalten.

7. Reinigungstuch gemäß Anspruch 1 bis 6, worin die Menge der Milchsäure-produzierenden Bakterien 10⁴-10¹¹ koloniebildende Einheiten ("colony forming units") (CFU) je Tuch beträgt.

8. Reinigungstuch gemäß Anspruch 7, worin die Menge der Milchsäure-produzierenden Bakterien 10⁶-10¹¹ koloniebildende Einheiten (CFU) je Tuch beträgt.

9. Feuchtigkeitsundurchlässige Verpackungseinheit, die ein Reinigungstuch gemäß Anspruch 1 bis 8 enthält, wobei die undurchlässige Verpackungseinheit eine höchste Wasserdampf-Transmissionsrate von 6 g/m²/Kalendertag in Übereinstimmung mit ASTME 398-83 aufweist.

## Revendications

1. Lingette hygiénique adaptée pour nettoyer et simultanément rétablir et maintenir une flore microbienne bénéfique sur la peau ou la zone urogénitale, ladite lingette hygiénique comprenant une matrice **caractérisée en ce que** ladite matrice est imprégnée par une composition constituée par
A) une préparation d'une ou de plusieurs souches bactériennes produisant de l'acide lactique, ladite préparation ayant une activité de l'eau de 0,30 ou moins, de manière davantage préférée de 0,25 ou moins, de manière préférée entre toutes de 0,20 ou moins ; et
B) une phase lipidique, de préférence une phase lipidique liquide, comprenant au moins un lipide, dans laquelle le lipide est choisi dans le groupe constitué par l'huile d'olive, l'huile de colza, l'huile de coco, l'huile de palmiste, l'huile d'arachide, l'huile de soja, la diméthicone, l'huile de paraffine, et la vaseline.

2. Lingette hygiénique selon la revendication 1, qui comprend en outre au moins un composant supplémentaire, choisi dans le groupe comprenant des agents de soin, des agents absorbant l'eau, des tensioactifs, des agents tamponnant le pH, un parfum, des antioxydants, de l'hydrocortisone et autres stéroïdes anti-inflammatoires.

3. Lingette hygiénique selon les revendications 1 et 2, dans laquelle la (les) souche(s) bactérienne(s) produisant l'acide lactique est (sont) isolée(s) de la peau ou de la zone urogénitale d'une personne saine.

4. Lingette hygiénique selon les revendications 1 à 3, dans laquelle la (les) souche(s) bactérienne(s) produisant l'acide lactique est (sont) choisie(s) parmi les genres *Pediococcus, Lactococcus, Lactobacillus,* ou les mélanges de ceux-ci.

5. Lingette hygiénique selon la revendication 4, dans laquelle la (les) souche(s) bactérienne(s) produisant l'acide lactique implique(nt) au moins *Lactobacillus plantarum.*

6. Lingette hygiénique selon la revendication 5, dans laquelle la (les) souche(s) bactérienne(s) produisant l'acide lactique implique(nt) au moins *Lactobacillus plantarum* 931 (n° de dépôt (DSM) : 11918).

7. Lingette hygiénique selon les revendications 1 à 6, dans laquelle la quantité de bactéries produisant de l'acide lactique est de 10⁴ à 10¹¹ unités formant colonies (UFC) par lingette.

8. Lingette hygiénique selon la revendication 7, dans laquelle la quantité de bactéries produisant de l'acide lactique est de 10⁶ à 10¹¹ unités formant colonies (UFC) par lingette.

9. Unité d'emballage imperméable à l'humidité contenant une lingette hygiénique selon les revendications 1 à 8, ladite unité d'emballage imperméable ayant un taux de transmission de la vapeur d'eau le plus élevé de 6 g/m²/jour calendaire selon ASTME 398-83.
